# EUROPÄISCHE PATENTANMELDUNG

(11) **EP 2 987 736 A1**
(43) Veröffentlichungstag der Anmeldung: **24.02.2016**
(21) Anmeldenummer: 15187403.9
(22) Anmeldetag: 12.08.2010
(51) Int. Cl.: B65B 55/02, B65B 55/14, A61J 1/05, A61J 1/06, A61F 9/00

(54) **VERFAHREN ZUR HERSTELLUNG EINES IN EINER SCHUTZHÜLLE EINGESCHWEISSTEN BEHÄLTNISSES**

(30) Priorität: 03.09.2009 AT 13922009
(62) Teilanmeldung aus: 10749591.3
(71) Anmelder: VALEANT sp. z o.o. sp. j., 35-959 Rzeszów (PL)
(72) Erfinder: Findl, Oliver, 1010 Wien (AT)
(74) Vertreter: Schwarz & Partner

(57) **Zusammenfassung**

Verfahren zur Herstellung eines in einer Schutzhülle eingeschweißten Behältnisses, welches ein viskoelastisches Fluid zur Verwendung als Augentropfen bei der chirurgischen Behandlung von Augen enthält, wobei das Behältnis derart gestaltet ist, dass das Fluid über eine Sollbruchstelle dem Behältnis entnommen werden kann, gekennzeichnet durch die Kombination der Maßnahmen, dass
- das viskoelastische Fluid in das Behältnis abgefüllt wird, wonach dieses verschlossen wird,
- das verschlossene Behältnis in einer Schutzhülle eingeschweißt wird, wonach
- das eingeschweißte Behältnis samt Schutzhülle einer thermischen Sterilisation unterzogen wird.

## Beschreibung

Die Erfindung betrifft ein Verfahren zur Herstellung eines in einer Schutzhülle eingeschweißten Behältnisses, welches ein Fluid enthält, das als Medizinprodukt in der Augenchirurgie verwendet wird. Die Erfindung betrifft ferner das in der Schutzhülle eingeschweißte Behältnis, welches nach dem erfindungsgemäßen Verfahren erhältlich ist.

Gewöhnlich wird das Auge während einer Operation in regelmäßigen Abständen mit einer Salzlösung befeuchtet, um ein Austrocknen der Cornea zu verhindern. Dieser Vorgang unterbricht jedoch die Arbeit des Operateurs, beeinträchtigt den operativen Fortgang und zerstört die Homöostase des Tränenfilms. In weiterer Folge werden dadurch wichtige Bestandteile des Tränenfilms wie z.B. entzündungshemmende Enzyme, Lipide und Mucopolysaccharide ausgewaschen.

Es ist ferner bekannt, in Spritzen abgefüllte viskoelastische Fluide, sogenannte Ophthalmic Viscosurgical Devices (OVD) auch zur anhaltenden Befeuchtung und zum Epithelschutz der Cornea während bestimmter Augenoperationen zu verwenden. Das Fluid ist z.B. eine viskoelastische, physiologisch verträgliche Formulierung, die zum Schutz der Cornea vor Austrocknung sowie vor Epithelschäden während Augenoperationen, wie Kataraktoperationen, Glaukomoperationen, Fremdkörperentfernung, oder Operationen des hinteren Augenbereichs (Hinterabschnittschirurgie wie z.B. Vitrektomie, Trabekulektomie), verwendet wird. Das Fluid wird hierbei aus einer Spritze ausgedrückt und - falls für die optimale Verteilung notwendig - mit einer Spatula oder Microsponge auf der Cornea verteilt. Das Fluid muss steril sein.

Ferner enthalten bekannte Fluide Konservierungsmittel, die jedoch für die medizinische Anwendung nachteilig sein können. Ferner sollte auch die außenseitige Oberfläche des Behältnisses, in welchem sich das Fluid befindet, steril sein, wodurch dem Chirurgen die Handhabung erleichtert wird.

Gemäß der US 2,813,623 sind elastische Kapseln die flüssige Medikamente enthalten beschrieben. Die beschriebenen Packungen werden dabei durch Bestrahlung sterilisiert.

In der EP 0 322 1345 sind eingeschweißte Fläschchen mit flüssigem Inhalt, der als Augentropfen verwendbar ist, beschrieben, wobei die Fläschchen einen Schraubverschluss besitzen, durch dessen Öffnung der Inhalt entnommen werden kann.

In der US 4,150,744 sind Behältnisse für Augentropfen beschrieben, die sich in einer Umhüllung befinden, in der eine sauerstofffreie Atmosphäre eingebracht ist. Diese Behältnisse besitzen einen Spender, mit dessen Hilfe gewünschte Portionen des Inhalts des Behältnisses abgegeben werden können, sowie eine Verschlusskappe, mit deren Hilfe der Behälter nach Abgabe der gewünschten Medikamentenportion wieder verschlossen werden kann.

In GB 1 275 903 sind Verpackungen geoffenbart, in denen ein Medikament in einem Behältnis mit entfernbarer (Verschluss)kappe vorliegt.

Verpackungen und deren Herstellung gemäß dem Stand der Technik entsprechen aber nicht immer in jeder Hinsicht den Anforderungen, die an Arzneimittelverpackungen, die ein viskoelastisches Fluid zur Verwendung bei der chirurgischen Behandlung von Augen enthalten und deren Herstellung gestellt werden.

Hier setzt nun die vorliegende Erfindung an, die sich zum Ziel setzt, die oben genannten Nachteile bzw. Unzulänglichkeiten zu beseitigen.

Das Ziel wird erreicht mit einem Verfahren zur Herstellung eines in einer Schutzhülle eingeschweißten Behältnisses, welches ein viskoelastisches Fluid zur Verwendung bei der chirurgischen Behandlung von Augen enthält, wobei das Behältnis derart gestaltet ist, dass das Fluid über eine Sollbruchstelle dem Behältnis entnommen werden kann, welches Verfahren gekennzeichnet ist durch die Kombination der Maßnahmen, dass
- das viskoelastische Fluid in das Behältnis abgefüllt wird, wonach dieses verschlossen wird,
- das verschlossene Behältnis in einer Schutzhülle eingeschweißt wird, wonach
- das eingeschweißte Behältnis samt Schutzhülle einer thermischen Sterilisation unterzogen wird.

Nach dem Einschweißen des Behältnisses in die Schutzhülle wird durch die terminale Sterilisation des Produktes die innere und äußere Sterilität des Produktes gewährleistet. Ein weiterer Vorteil des erfindungsgemäßen Verfahrens besteht darin, dass dem viskoelastischen Fluid kein Konservierungsmittel zugegeben werden braucht.

Das erfindungsgemäß hergestellte Behältnis gewährleistet einen höheren Komfort für den Chirurgen bei der Verwendung, sowie eine höhere Sicherheit beim Patienten.

Eine bevorzugte Ausführungsform des erfindungsgemäßen Verfahrens besteht darin, dass das Behältnis ein Einzeldosenbehältnis ist.

Das Behältnis bzw. Einzeldosenbehältnis ist bevorzugt aus Polypropylen oder Mischungen von Polyethylen oder Polypropylen mit Copolymeren aus Ethylen und Propylen oder aus einem Laminat.

Die Schutzhülle besteht vorzugsweise aus einem sterilisierbaren Medizinalpapier und einer Verbundfolie (z.B. Medipeel® Pouch von Sengewald) oder **Tyvek**^{®} Material (Hersteller DuPont).

Die thermische Sterilisation kann bei einer Temperatur zwischen 80 und 140°C vorgenommen werden.

Die Erfindung betrifft ferner das nach dem erfindungsgemäßen Verfahren herstellbare, in einer Schutzhülle eingeschweißte Behältnis als solches.

### Beschreibung der Formulierung

Die Formulierung enthält mindestens ein viskositätserhöhendes, physiologisch verträgliches Polymer, das bekannterweise die Augenoberfläche befeuchtet, wie zum Beispiel

Hydroxypropylmethylzellulose, Carboxymethylzellulose, Hydroxyethylzellulose, Hyaluronsäure, Natriumalginat, Hydroxylpropyl-Guar, Polyvinylpyrrolidon (Povidon), Polyvinylalkohol, Polymethacrylsäure (Carbomer), Polyethylenglykol.

Die Konzentration beträgt vorzugsweise 0,01-10%.

Ferner können auch Kombinationen von zwei oder mehreren dieser Polymere verwendet werden.

Da das Fluid am Auge eingesetzt wird, sollte die Zusammensetzung vorzugsweise einen pH-Wert von 6 bis 8,5, vorzugsweise 6,5 bis 8, noch mehr bevorzugt 6,8 bis 7,6, aufweisen.

Bei der Verabreichung von Zusammensetzungen am Auge ist es weiter von Vorteil, wenn diese eine der Tränenflüssigkeit vergleichbare Osmolarität aufweisen. Daher beträgt die Osmolarität der erfindungsgemäßen Zusammensetzung vorzugsweise 200 bis 400 mosmol/l, noch mehr bevorzugt 250 bis 330 mosmol/l.

Die dabei benötigten Hilfsstoffe wie z.B. Puffersalze, Stabilisatoren, Hilfsstoffe zur Einstellung der gewünschten Osmolalität und Hilfsstoffe zur Erhöhung der Verträglichkeit hängen jeweils von der Formulierung ab und sind dem Fachmann hinreichend bekannt.

### Beschreibung der Verpackung

Die Einzeldosenbehältnisse bestehen vorzugsweise aus Polypropylen (PP) in pharmazeutischer Qualität. Der für die Herstellung der Einzeldosenbehältnisse bevorzugt verwendete Polypropylen Rohstoff weist folgende Eigenschaften auf:
Schmelzpunkt (bestimmt nach ISO 3146): 100°C - 260°C
Vicat-Erweichungstemperatur (10N, 50°C pro Stunde; bestimmt nach ISO 306): 80°C - 240°C;
Schmelzflussindex (230°C / 2,16kg; bestimmt nach ISO 1133): 0,1g/10 min - 50g/10 min;
Streckdehnung (50mm/min; bestimmt nach ISO 527-2): 1% - 30%;
Charpy-Kerbschlagzähigkeit (bei 23°C; bestimmt nach ISO 179): 1 kj/m² - 20 kj/m²

Die Schutzhülle für das Einzeldosenbehältnis besteht vorzugsweise aus einem sterilisierbaren Medizinalpapier und einer Spezialverbundfolie, wobei eine Seite durchsichtig ist (z.B. Medipeel^{®}Pouch von Sengewald) und gewährleistet äußere Sterilität des Einmaldosenbehältnisses.

### Beschreibung der Sterilisation

Durch die spezielle Verpackung des viskoelastischen Fluides in einem Einzeldosenbehältnis und einer darüberliegenden Schutzhülle wird somit in einem einzigen - terminalen - Sterilisationsschritt die innere und äußere Sterilität des Produktes gewährleistet. Die bevorzugte Art der Sterilisation ist die physikalische Sterilisation durch Hitze in einem Temperaturbereich von 80°C-140°C z.B. durch Heisswasserberieselung, Sattdampfsterilisation oder Sterilisation mit einem Dampf-Luft-Gemisch.

Es hat sich überraschend gezeigt, dass die terminale Sterilisation mit ionisierenden Strahlen im vorliegenden Fall nicht geeignet ist, da es zum unkontrollierten Abbau der viskositätserhöhenden Polymere im Fluid kommt und das Produkt nach der Sterilisation somit die erforderlichen viskoelastischen Eigenschaften nicht mehr aufweist. Alle anderen Sterilisationsmethoden haben den Nachteil, dass die Sterilisation nicht als terminale Sterilisation im Endbehältnis erfolgen kann, sondern zwei Sterilisationsschritte für innere and äußere Sterilität erforderlich wären.

### Beispiel für ein viskoelastisches Fluid

- Wasser f. Injektionszwecke
- Natriumhydroxid
- Milchsäure
- Natriumchlorid
- Kaliumchlorid
- Calciumchlorid x 2H2O
- Hydroxypropylmethylzellulose
Füllvolumen: 2 ml
pH: 6,8-7,6
Osmolalität: 265-330 mosmol/kg

Die Erfindung betrifft weiters:
Ein
   1. Verfahren zur Herstellung eines in einer Schutzhülle eingeschweißten Behältnisses, welches ein viskoelastisches Fluid zur Verwendung als Augentropfen bei der chirurgischen Behandlung von Augen enthält, wobei das Behältnis derart gestaltet ist, dass das Fluid über eine Sollbruchstelle dem Behältnis entnommen werden kann, gekennzeichnet durch die Kombination der Maßnahmen, dass
      - das viskoelastische Fluid in das Behältnis abgefüllt wird, wonach dieses verschlossen wird,
      - das verschlossene Behältnis in einer Schutzhülle eingeschweißt wird, wonach
      - das eingeschweißte Behältnis samt Schutzhülle einer thermischen Sterilisation unterzogen wird.
   2. Verfahren nach Punkt 1, dadurch gekennzeichnet, dass das Behältnis ein für die Applikation von Augentropfen geeignetes Einzeldosenbehältnis ist.
   3. Verfahren nach einem der Punkte 1 oder 2, dadurch gekennzeichnet, dass das Behältnis bzw. Einzeldosenbehältnis aus Polypropylen oder Mischungen von Polyethylen oder Polypropylen mit Copolymeren aus Ethylen und Propylen oder aus einem Laminat ist.
   4. Verfahren nach einem der Punkte 1 bis 3, dadurch gekennzeichnet, dass die Schutzhülle aus einem sterilisierbaren Medizinalpapier und einer Verbundfolie oder Tyvek^{®} Material besteht.
   5. Verfahren nach einem der Punkte 1 bis 4, dadurch gekennzeichnet, dass die thermische Sterilisation bei einer Temperatur zwischen 80 und 140°C vorgenommen wird.
   6. Verfahren nach einem der Punkte 1 bis 5, dadurch gekennzeichnet, dass das Fluid frei von Konservierungsmittel ist.
   7. Ein in einer Schutzhülle eingeschweißtes Behältnis, erhältlich nach einem Verfahren gemäß einem der Punkte 1 bis 6.

## Patentansprüche

1. Verfahren zur Herstellung eines in einer Schutzhülle eingeschweißten Behältnisses, welches ein viskoelastisches Fluid zur Verwendung als Augentropfen bei der chirurgischen Behandlung von Augen enthält, wobei das Behältnis derart gestaltet ist, dass das Fluid über eine Sollbruchstelle dem Behältnis entnommen werden kann, **gekennzeichnet durch** die Kombination der Maßnahmen, dass
- das viskoelastische Fluid in das Behältnis abgefüllt wird, wonach dieses verschlossen wird,
- das verschlossene Behältnis in einer Schutzhülle eingeschweißt wird, wonach
- das eingeschweißte Behältnis samt Schutzhülle einer thermischen Sterilisation unterzogen wird.

2. Verfahren nach Anspruch 1, **dadurch gekennzeichnet, dass** das Behältnis ein für die Applikation von Augentropfen geeignetes Einzeldosenbehältnis ist.

3. Verfahren nach einem der Ansprüche 1 oder 2, **dadurch gekennzeichnet, dass** das Behältnis bzw. Einzeldosenbehältnis aus Polypropylen oder Mischungen von Polyethylen oder Polypropylen mit Copolymeren aus Ethylen und Propylen oder aus einem Laminat ist.

4. Verfahren nach einem der Ansprüche 1 bis 3, **dadurch gekennzeichnet, dass** die Schutzhülle aus einem sterilisierbaren Medizinalpapier und einer Verbundfolie oder Tyvek^{®} Material besteht.

5. Verfahren nach einem der Ansprüche 1 bis 4, **dadurch gekennzeichnet, dass** die thermische Sterilisation bei einer Temperatur zwischen 80 und 140°C vorgenommen wird.

6. Verfahren nach einem der Ansprüche 1 bis 5, **dadurch gekennzeichnet, dass** das Fluid frei von Konservierungsmittel ist.

7. Verfahren nach einem der Ansprüche 1 bis 6, **dadurch gekennzeichnet, dass** das Fluid frei von Konservierungsmittel ist.

8. Verfahren nach einem der Ansprüche 1 bis 7, **dadurch gekennzeichnet, dass** das viskoelastische Fluid zumindest ein Polymer ausgewählt aus der Gruppe Hydroxypropylmethylzellulose, Carboxymethylzellulose, Hydroxyethylzellulose, Hyaluronsäure, Natriumalginat, Hydroxylpropyl-Guar, Polyvinylpyrrolidon (Povidon), Polyvinylalkohol, Polymethacrylsäure (Carbomer), Polyethylenglykol aufweist.

9. Viskoelastisches Fluid zur Verwendung als Augentropfen bei der chirurgischen Behandlung von Augen, wobei das viskoelastische Fluid in einem verschlossenen Behältnis angeordnet ist, welches in einer Schutzhülle eingeschweißt ist, wobei das Behältnis ein Einzeldosenbehältnis ist, welches derart gestaltet ist, dass das Fluid über eine Sollbruchstelle dem Behältnis entnommen werden kann, wobei das eingeschweißte Behältnis innen und außen steril ist.

10. Viskoelastisches Fluid zur Verwendung als Augentropfen nach Anspruch 9, **dadurch gekennzeichnet, dass** es zumindest ein Polymer ausgewählt aus der Gruppe Hydroxypropylmethylzellulose, Carboxymethylzellulose, Hydroxyethylzellulose, Hyaluronsäure, Natriumalginat, Hydroxylpropyl-Guar, Polyvinylpyrrolidon (Povidon), Polyvinylalkohol, Polymethacrylsäure (Carbomer), Polyethylenglykol aufweist.

11. Viskoelastisches Fluid zur Verwendung als Augentropfen nach Anspruch 9 oder Anspruch 10, **dadurch gekennzeichnet, dass** die Schutzhülle aus einem sterilisierbaren Medizinalpapier und einer Verbundfolie oder Tyvek^{®} Material besteht.

12. Einzeldosenbehältnis, umfassend ein viskoelastisches Fluid zur Verwendung als Augentropfen bei der chirurgischen Behandlung von Augen, wobei das Einzeldosenbehältnis derart gestaltet ist, dass das Fluid über eine Sollbruchstelle entnommen werden kann, wobei das Einzeldosenbehältnis innen und außen steril ist und wobei das Einzeldosenbehältnis in einer Schutzhülle eingeschweißt ist.

13. Einzeldosenbehältnis nach Anspruch 12, **dadurch gekennzeichnet, dass** das viskoelastisches Fluid ein Polymer ausgewählt aus der Gruppe Hydroxypropylmethylzellulose, Carboxymethylzellulose, Hydroxyethylzellulose, Hyaluronsäure, Natriumalginat, Hydroxylpropyl-Guar, Polyvinylpyrrolidon (Povidon), Polyvinylalkohol, Polymethacrylsäure (Carbomer), Polyethylenglykol aufweist.

14. Einzeldosenbehältnis nach Anspruch 12 oder Anspruch 13, **dadurch gekennzeichnet, dass** die Schutzhülle aus einem sterilisierbaren Medizinalpapier und einer Verbundfolie oder Tyvek^{®} Material besteht.
